# EUROPEAN PATENT SPECIFICATION

(11) **EP 1 771 220 B1**
(45) Date of publication and mention of the grant of the patent: **14.11.2018**
(21) Application number: 05785445.7
(22) Date of filing: 14.07.2005
(51) Int. Cl.: A61M 16/00, F16K 31/02, A62B 7/00

(54) **APPARATUS FOR NON-REBREATHING POSITIVE AIRWAY PRESSURE VENTILATION**
VORRICHTUNG ZUR NICHTRÜCKATMUNGS-ÜBERDRUCKBEATMUNG
APPAREIL DE VENTILATION PAR PRESSION POSITIVE SANS RE-INSPIRATION

(30) Priority: 14.07.2004 US 587781 P; 08.07.2005 US 178052
(43) Date of publication of application: 11.04.2007
(73) Proprietor: RIC Investments, LLC., Wilmington, DE 19801-1545 (US)
(72) Inventor: ZARYCHTA, Jaroslaw, Winnipeg, Manitoba R3R 1A6 (CA)
(74) Representative: de Haan, Poul Erik
(86) International application number: PCT/US2005/025319
(87) International publication number: WO 2006/020146

(56) References cited:
- EP-A2- 0 714 670
- WO-A2-2004/047621
- US-A- 6 105 575
- US-B1- 6 968 842
- US-B2- 6 948 497

## Description

### TECHNICAL FIELD

The present disclosure relates to method of ventilating a patient, and, in particular, to a method of ventilating a patient that provides improved fluid exchange in the lungs, and the present invention
to a medical ventilator that implements such a mode of ventilation.

### BACKGROUND OF THE INVENTION

It is the goal of medical ventilation to safely and effectively ventilate a patient in accordance with the patient's physiological needs. Accordingly, various methods of operating a medical ventilator in accordance with the various needs of patients have been devised. For example, it is well known to ventilate a patient such that a desired pressure, flow, or volume of fluid is delivered to the patient during inspiration and fluid is allowed to exhaust from the patient during expiration. Conventional ventilator provide a variety of modes for ventilating a patient and allow control over the trigger, the cycle, and the limit for the delivery of fluid to the patient. U.S. Patent No. 5,868,133 to DeVries discusses such conventional ventilators and their operation. Document EP0714670 describes a method of detecting changes in the respiratory state of a patient between inspiration and expiration. Document WO2004/047621 describes yet another apparatus for delivering pressurized breathing gas to an airway of a patient characterized in that the processing means determines a pressure to be delivered to a patient during inhalation and a pressure to be delivered to such a patient during exhalation

In the treatment of acute lung injuries or adult respiratory distress syndrome (ARDS), a mode of ventilation known as airway pressure release ventilation (APRV), described hereinafter, has gained acceptance. This mode of ventilation is similar to a continuous positive airway pressure (CPAP) mode of pressure support in which a flow of fluid at a constant pressure is delivered to the airway of the patient, with regular, brief, intermittent releases in airway pressure imposed on the CPAP pressure. Although the APRV mode of ventilation has gained acceptance in many circles, the conventional mode of ventilation, which is also described hereinafter, is still preferred, because it is more natural to patients than the APRV mode of ventilation.

What is needed, however, and not found in the prior art, is a mode of ventilation, and a medical ventilator that implements a mode of ventilation that combines the advantages of the conventional mode of ventilation and the APRV mode of ventilation in a manner that minimizes patient discomfort, while, at the same time, increases the amount of fluid, i.e., gas or liquid, exchanged from the patient's lungs and increases the amount of fresh fluid introduced into the patient's lungs during each ventilator cycle. Document US 6,105,575 discloses a medical ventilator according to the preamble of claim

### 1. DISCLOSURE OF THE INVENTION

Accordingly, it is an object of the present disclosure to provide a method of ventilating a patient that satisfies this need. This object is achieved by providing a method of ventilating a patient that includes (a) delivering fluid to a patient during an exhalation/quiescent phase, in which a pressure, a flow, or a volume of fluid is provided at a baseline level, (b) releasing the flow of fluid from such a patient during a release phase following the exhalation/quiescent phase, in which the pressure, flow, or volume of fluid is decreased from the baseline level to a release level that is less than the baseline level. After the release phase, the pressure, flow, or volume of fluid delivered to such a patient is increased during a delivery phase to a peak level above the baseline level. Thereafter, the pressure, flow, or volume of fluid is allowed to return from the peak level to the baseline level. The present invention proposes a medical ventilator according to claim 1.

It is a further object of the present invention to provide a medical ventilator that includes a patient circuit, a pressure generator for delivering pressurized fluid to a patient via the patient circuit, and means for controlling the pressure generator. More specifically, the pressure generator is controlled by the controlling means so as to supply the flow of fluid to the patient in accordance with the method set forth above.

It is a still further object of the present invention to provide a system for ventilating a patient according to a method that includes (a) providing a fluid to a patient at a first pressure; (b) providing the fluid to the patient at a second pressure after terminating the provisioning of the fluid at the first pressure, wherein the second pressure is less than the first pressure; and (c) providing the fluid to the patient at a third pressure after terminating the provisioning of the fluid at the second pressure, wherein the third pressure is greater than the first pressure. Steps (a) through (c) are repeated over each respiratory cycle.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention. As used in the specification and in the claims, the singular form of "a", "an", and "the" include plural referents unless the context clearly dictates otherwise.

### BRIEF DESCRIPTION OF THE DRAWINGS

FIG. 1 is a schematic block diagram of ventilation system, including a patient, a patient circuit, and a ventilator operative for implementing a mode of ventilation in accordance with the principles of the present invention;
FIGS. 2A-2C are waveforms associated with ventilating a patient in a conventional mode of ventilation;
FIGS. 3A-3C are waveforms associated with ventilating a patient in an airway pressure release ventilation (APRV) mode of ventilation;
FIGS. 4A-4C are waveforms associated with ventilating a patient in accordance with the present invention; and
FIGS. 5-7 are pressure waveforms illustrating triggering and cycling techniques using the ventilation mode of the present invention.

### BEST MODE(S) FOR CARRYING OUT THE INVENTION

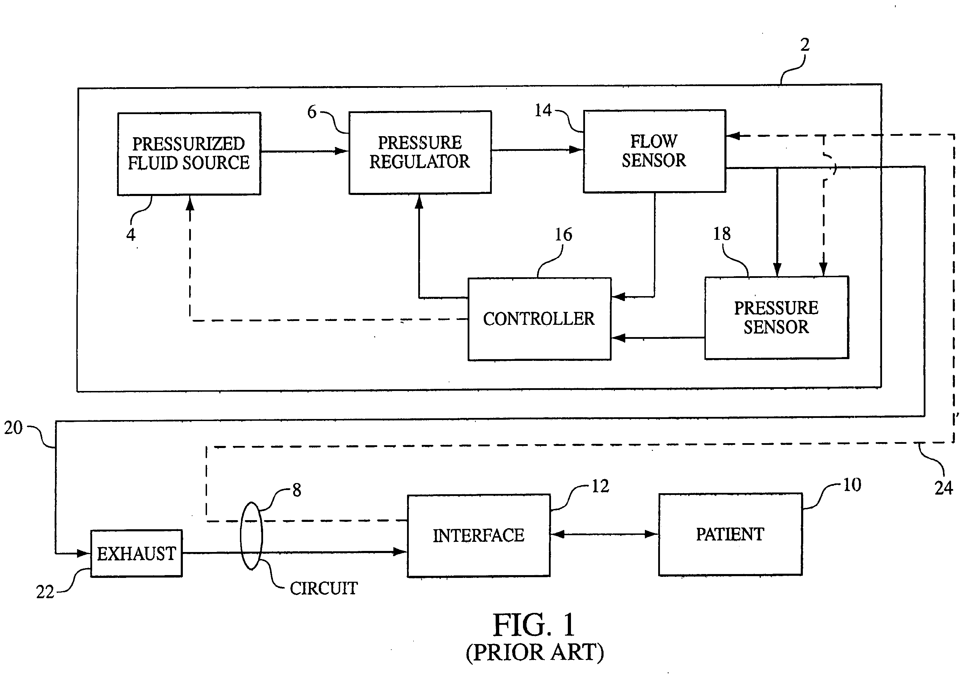

With reference to FIG. 1, a medical ventilator 2 typically includes a pressurized fluid source 4 and a pressure regulator 6 connected to receive pressurized fluid from pressurized fluid source 4. The source of pressurized fluid can be gas from a conventional pressurized tank, gas in which the pressure is elevated by a pressure generator, such as bellows, piston, or blower, or a combination thereof. Pressure regulator 6 regulates the pressure of the pressurized fluid, i.e., a gas or a liquid, supplied to a circuit 8, which conveys the pressure regulated fluid to a patient 10 via an interface 12. It can be appreciated that the pressurized fluid source and the pressure regulator can be combined into a common component that is collectively referred to as the pressure generator. For example, it is known to control the operation of the blower or compressor to produce a desired output pressure for the flow of gas exiting the pressure generating system in combination with a gas flow/pressure control valve either upstream or downstream of the blower or compressor. As used herein, the term "ventilator" or "ventilation system" refers to a device or system that delivers a flow of pressurized fluid to an airway of a patient. These terms include life support ventilators (operated either invasively or non-invasively) and a pressure support system, such as a CPAP, a bilevel system, which delivers a flow of gas at an inspiratory positive airway pressure (IPAP) during inspiration and an expiratory positive airway pressure (EPAP) during expiration.

Medical ventilator 2 includes a flow sensor 14 for detecting a flow and/or volume of fluid supplied to circuit 8 from pressure regulator 6, and to provide to a controller 16 a signal indicative of the detected fluid flow, volume, or both. If desired, however, flow sensor 14 can be omitted.

A pressure sensor 18 is also typically provided to detect a pressure of the flow of fluid in circuit 8 and, more particularly, at interface 12. The pressure signal from sensor 18 is provided to controller 16, where can be used to monitor the pressure of the flow of fluid delivered to the patient, and, in particular, to detect patient inhalation and exhalation. In response to the one or more signals from flow sensor 14, pressure sensor 18, or both, controller 16 causes pressure regulator 6 to supply pressurized fluid to patient 10 in the manner described in detail hereinafter. Alternatively, as shown by the dashed line between controller 16 and pressurized fluid source 4, controller 16 can control pressurized fluid source 4 to supply pressurized fluid to patient 10.

As noted above, pressurized fluid source 4 can include a source, e.g., a piston, a bellows or a blower, of fluid, e.g., breathing gas, gas, air, oxygen, helium-oxygen or breathing liquid. Pressure regulator 6 can include a poppet valve, solenoid, butterfly valve or sleeve valve. If pressurized fluid source 4 includes a piston or a blower, controller 16 can control the pressure and/or flow of fluid from pressurized fluid source 4 by controlling the speed of the piston or the speed of the blower.

In one embodiment of the present invention, circuit 8 includes a first tube or conduit 20 connected between pressure regulator 6 and interface 12, i.e., a single-limb circuit. First tube or conduit 20 includes an exhaust assembly 22, which can be an active exhaust or a passive exhaust. The active exhaust can include a modified poppet valve that operates to block or limit the flow of exhaust gas from the patient circuit via exhaust assembly 22 when the flow of fluid is supplied to patient 10 during inhalation and to open or increase the flow of exhaust gas from the patient circuit when patient 10 exhales. The passive exhaust can be a hole or other aperture defined in first tube or conduit 20, in patient interface 12, or both. The size, shape, number, and location of the hole or holes can be selected as a compromise between enabling patient 10 to exhale naturally so as to remove exhaust gasses from the patient circuit, and enabling the flow of fluid to be supplied to patient 10 during inhalation.

Alternatively, circuit 8 can exclude exhaust 22 and can include a second tube or conduit 24, i.e., a two-limb circuit, wherein second tube or conduit 24 enables fluid exhaled by patient 10 to be conveyed to ventilator 2, where the exhaled fluid is exhausted to atmosphere. Typically, an exhaust flow control system is provided in the ventilator that controls the exhausting of fluid from the second limb.

Although not shown, it is also known to provide a supplemental gas, such as oxygen, to the patient circuit. The source of supplemental gas can be any conventional gas source, such as an oxygen tank, liquid oxygen storage vessel, hospital oxygen wall supply, or oxygen concentrator. The flow of supplemental gas can be introduced into the primary gas flow at any location, including in the ventilator, upstream or downstream of the pressurized fluid source, the pressure regulator, the flow sensor, or the pressure sensor. The flow of supplemental gas can also be introduced into conduit 24 or interface 12.

Two prior art modes of ventilating patient 10, namely a conventional mode and an APRV mode, utilizing ventilator 2 will now be described, followed by a description of a mode of ventilating patient 10 utilizing ventilator 2 in accordance with the present invention, namely an NRPAP mode. In the following descriptions, ventilation of patient 10 will be described as occurring in synchronization with the breathing or breath cycle of the patient. However, this is not to be construed as limiting the invention, because the ventilation of patient 10, especially in the APRV and NRPAP modes of operation, can occur independent of the patient's breathing or breath cycle. Ventilation of patient 10 independent of the patient's breathing or breath cycle is often referred to in the art as "mandatory breathing" or "machine timed breathing". However, this is not to be construed as limiting the invention.

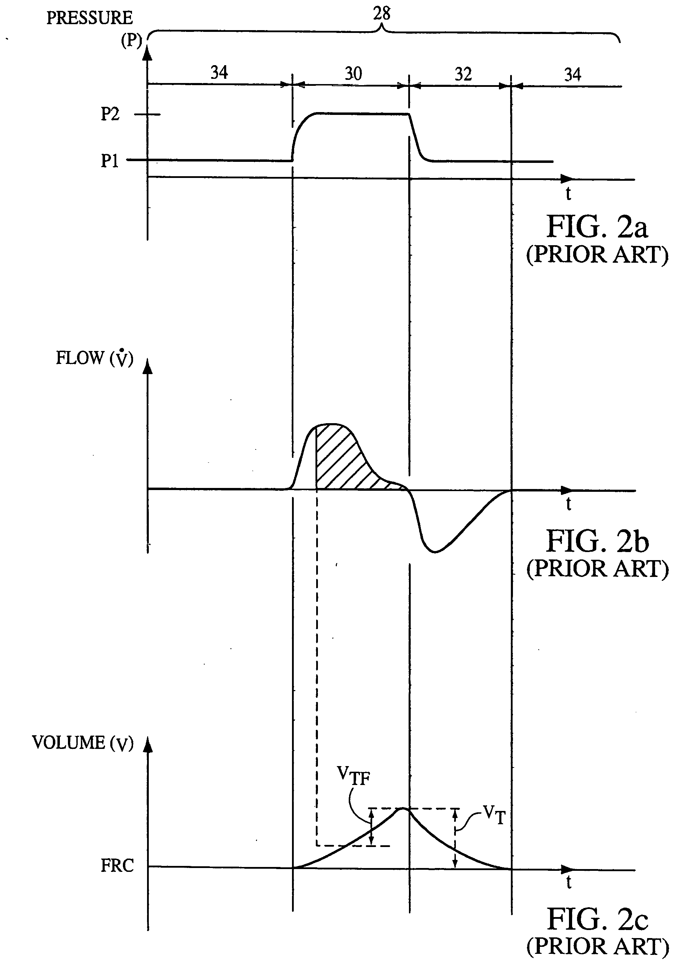

With reference to FIGS. 2A-2C and with continuing reference to FIG. 1, when operated in a conventional assist-control mode that is pressure limited, ventilator 2 causes the flow of fluid to be provided to patient 10 at a pressure P2 during a first period 30 and causes the flow of fluid to be provided to patient 10 at a pressure P1, less than pressure P2, during a second period 32 and a third period 34 of each ventilation cycle 28 applied to patient 10. In this conventional mode of ventilation, which is patient triggered, first period 30 of ventilation cycle 28 coincides with inhalation by patient 10, second period 32 coincides with exhalation by patient 10, and third period 34 coincides with a quiescent period of breathing by patient 10.

In FIGS. 2A-2C, third period 34 is shown on either side of first period 30 and second period 32 for illustration purposes in order for the latter two periods to be in the central part of the figure. However, this is not to be construed as limiting the invention since, as one skilled in the art would appreciate, the relative position of periods 30-34 in each x-y chart of FIGS. 2A-2C can be adjusted as desired. For example, the beginning of first period 30, second period 32 or third period 34 (immediately following second period 32) can be positioned on the ordinate axis of each x-y chart of FIGS. 2A-2C.

As shown in FIG. 2A, during third period 34 leading up to first period 30, the flow of fluid is provided to patient 10 at pressure P1, desirably a pressure between 0-10 centimeters of water (cm H₂O) (10.1973 cm H₂O = 1000 Pascals = 1000 N/m²). At or near the onset of first period 30, ventilator 2 increases the pressure of the flow of fluid from pressure P1 to pressure P2, desirably between 10-60 cm H₂O. At or near the onset of second period 32, ventilator 2 decreases the pressure of the flow of fluid from pressure P2 back to pressure P1. Thereafter, the pressure of the flow of fluid is maintained at pressure P1 until at or near the onset of the next first period 30. The process of changing from pressure P1 to pressure P2 and then back to pressure P1 in this conventional mode of operation of ventilator 2 is desirably repeated for each breath cycle of patient 10 according to when patient 10 commences inhaling during each first period 30 and exhaling during each second period 32. However, this is not to be construed as limiting the invention.

FIG. 2B illustrates the fluid flow (velocity of fluid) entering patient 10 during first period 30, the fluid flow exiting patient 10 during second period 32, and the absence of fluid flow entering or exiting patient 10 during third period 34. The area shown in crosshatch represents the fresh fluid that flows into the patient's lungs during first period 30.

FIG. 2C illustrates the volume of fluid that enters and exits patient 10 during first period 30 and second period 32, respectively, as well as the absence of fluid entering or exiting patient 10 during third period 34. In FIG. 2C, V_{T} represents the overall tidal volume of fluid entering and exiting the patient's lungs during each breath cycle, while V_{TF} represents the fresh fluid volume entering the patient's lungs during inhalation first period 30. V_{TF} corresponds to the crosshatched area in FIG. 2B. V_{T} is greater than V_{TF} because some of the volume in V_{T} is used to ventilate the dead space of the total patient respiratory system.

While FIGS. 2A-2C illustrate a pressure limited mode of conventional ventilation, it is to be understood that other conventional modes of ventilation are known. For example, the flow of fluid to the airway of a patient during inhalation can be limited based on the flow rate or volume. In addition, the triggering and cycling can be based on patient effort or it can be time based.

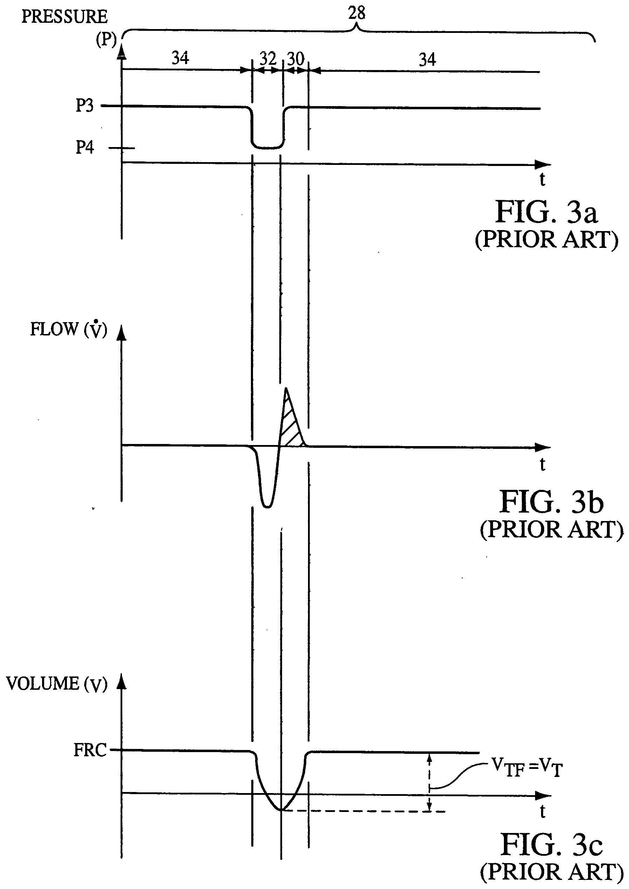

With reference to FIGS. 3A-3C and with continuing reference FIGS. 1 and 2A-2C, when operated in an airway pressure release ventilation (APRV) mode, ventilator 2 causes the flow of fluid to be provided to patient 10 at a pressure P3 during first period 30 and during third period 34 of each ventilation cycle 28 applied to patient 10. However, during second period 32 of each ventilation cycle 28, ventilator 2 causes the flow of fluid to be provided to patient 10 at a pressure P4 which is less than pressure P3.

In FIGS. 3A-3C, third period 34 is shown on either side of first period 30 and second period 32 for illustration purposes only and is, therefore, not to be construed as limiting the invention for at least the reason discussed above for third period 34 in FIGS. 2A-2C. The APRV mode of ventilation is described, for example, in U.S. Patent No. 4,773,411 to Downs.

As shown in FIG. 3A, during third period 34 leading up to second period 32, the flow of fluid is provided to patient 10 at pressure P3, desirably a pressure between 20-40 cm H₂O. At or near the onset of second period 32, ventilator 2 decreases the pressure of the flow of fluid from pressure P3 to pressure P4, desirably between 0-10 cm H₂O. At or near the onset of first period 30, ventilator 2 increases the pressure of the flow of fluid from pressure P4 back to pressure P3. Thereafter, the pressure of the flow of fluid is maintained at pressure P3 until at or near the onset of the next second period 32. The process of changing from pressure P4 to pressure P3 and then back to pressure P3 in the APRV mode of operation of ventilator 2 is repeated for each ventilation cycle 28 of patient 10 which desirably occurs independent of each breath cycle of patient 10, i.e., machine timed breathing.

If desired, however, each ventilation cycle 28 in the APRV mode of operation can be synchronized with each breath cycle of patient 10 whereupon, it is envisioned that patient 10 would exhale during second period 32 and/or just prior to the onset of second period 32, and inhale during first period 30. However, this is not to be construed as limiting the invention.

In the APRV mode of operation, the onset of second period 32 is delayed until just before the next first period 30. This is in contrast to the conventional mode of operation shown in FIG. 2B, wherein second period 32 immediately follows first period 30.

Comparing FIGS. 2C and 3C, it can be seen that the patient's functional residual capacity (FRC), in the APRV mode of operation is higher or greater than the patient's FRC in the conventional mode of operation. In contrast to FIG. 2C, where the volume of fluid in the patient's lungs returns to FRC during third period 34, in the APRV mode of operation shown in FIG. 3C, maintaining the flow of fluid at pressure P3 after first period 30 increases the patient's FRC. Thus, except during first and second periods 30 and 32 in the APRV mode of operation, the volume of fluid in the lungs of patient 10 is maintained at the higher FRC than the patient's FRC in the conventional mode of operation.

The area shown in crosshatch in FIG. 3B represents the velocity of fluid flow entering patient 10 during first period 30. V_{TF} in FIG. 3C represents the fresh fluid volume entering the patient's lungs during first period 30 and corresponds to the crosshatched area in FIG. 3B. Comparing FIGS. 2C and 3C, it can be seen that V_{TF} in the APRV mode of operation is greater than V_{TF} in the conventional mode of operation, and that V_{TF} equals V_{T} in the APRV mode of operation. Thus, in the APRV mode of operation more fluid will be exchanged in the lungs of patient 10 during each ventilation cycle 28 than in the conventional mode of operation.

Because the flow of fluid is provided to patient 10 at pressure P3 during first period 30 and third period 34, and because subjecting patient 10 to the flow of fluid at pressure P4 during third period 34 may not be comfortable for some patients 10, it may be necessary to sedate patient 10 when ventilator 2 is operated in the APRV mode of operation, especially when ventilator 2 utilizes machine timed breathing, to avoid patient discomfort. Notwithstanding, it has been observed that the APRV mode of operation is clinically significant in the treatment of certain respiratory diseases or disorders.

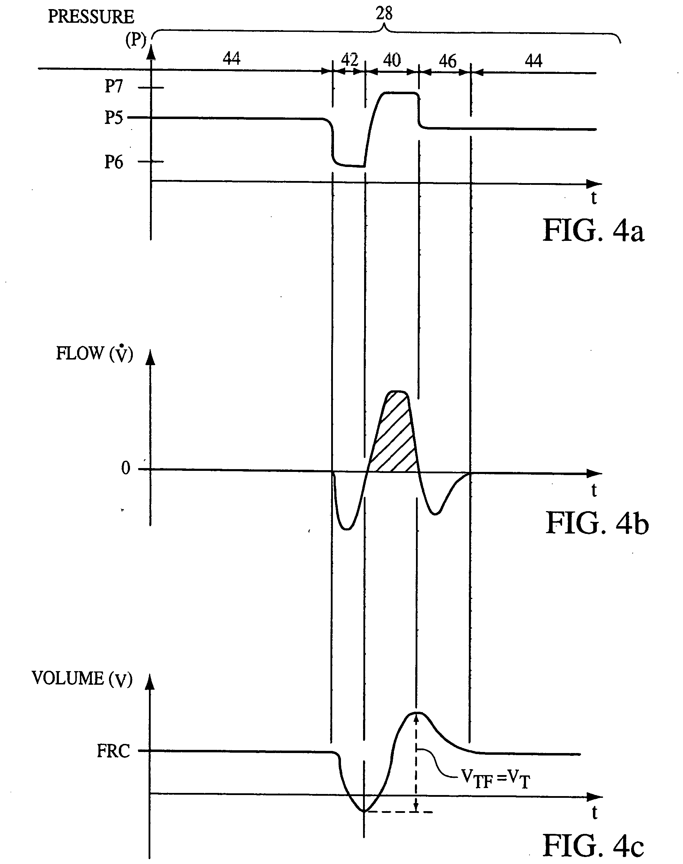

With reference to FIGS. 4A-4C, a Non-Rebreathing Positive Airway Pressure (NRPAP) mode of operation in accordance with the principles of the present invention will now be described. In the NRPAP mode of operation, ventilator 2 causes flow of fluid to be provided to patient 10 at a pressure P5 during a quiescent phase 44 of each ventilation cycle 28. The quiescent phase corresponds to a state of ventilation in which the pressure, flow, or volume of fluid delivered to the patient is maintained at a baseline level P5. In the illustrated exemplary embodiment, baseline level P5 corresponds to ventilating the patient such that the pressure, flow, or volume of fluid delivered to the patient is substantially constant.

The quiescent phase can be thought of as an extension of an exhalation phase 46. It can also be appreciated that the quiescent phase may be very short or even not present for some patient and under certain circumstances. That is, the quiescent phase can be replaced by the exhalation phase in cases when there is no clear period of constant pressure, flow, or volume of fluid at the end of an exhalation phase, e.g., during rapid breathing. Thus, the present invention refers to the period between a delivery phase 40 and release phase 42 collectively as an exhalation/quiescent phase, in which the presence of the quiescent phase may or may not be present.

During a release phase 42 of each ventilation cycle 28, ventilator 2 allows the flow of fluid to be released from the patient therefore removing the used gas from the patient's respiratory system that results in elimination or reduction of the dead space of the total patient respiratory system, i.e., patient lungs, airways, and part of the patient circuit. This is accomplished, for example, by reducing the pressure of the flow of fluid provided to patient 10 to a release pressure level P6 that is less than baseline pressure level P5. During a delivery phase 40 of each ventilation cycle 28, ventilator 2 delivers the flow of fresh fluid to the patient by increasing the pressure of the flow of fluid from the release pressure level P6 to a peak pressure level P7 that is greater than baseline level P5. Thereafter, the flow of fluid is controlled so as to allow the pressure, the flow, or the volume of fluid to return from peak level P7 to the baseline level P5 during an exhalation phase 46.

During the quiescent phase 44, which, as noted above, can be considered an extension of exhalation phase 46, ventilator 2 causes the flow of fluid to be provided to patient 10 at baseline pressure level P5. In FIGS. 4A-4C, quiescent phase 44 is shown on either side of release phase 42, delivery phase 40, and exhalation phase 46 for illustration purposes only, and is, therefore, not to be construed as limiting the invention for at least the reason discussed above for third period 34 in FIGS. 2A-2C.

As shown in FIG. 4A, during quiescent phase 44 leading up to release phase 42, the flow of fluid is provided to patient 10 at baseline pressure P5, desirably between 5-30 cm H₂O, typically about 15 cm H₂O. The present invention contemplates that baseline pressure P5 is set to the positive end expiratory pressure (PEEP) or is set based on the PEEP, such as PEEP plus a constant. At or near the onset of release phase 42, ventilator 2 decreases the pressure of the flow of fluid from pressure P5 to pressure P6, desirably between 0-15 cm H₂O, typically about 5 cm H₂O. At or near the onset of delivery phase 40, ventilator 2 increases the pressure of the flow of fluid from pressure P6 to pressure P7, desirably between 15-60 cm H₂O, typically about 30 cm H₂O. At or near the onset of exhalation phase 46 after delivery phase 40, ventilator 2 decreases the pressure of the flow of fluid from pressure P7 back to pressure P5. Thereafter, the pressure of the flow of fluid is maintained at pressure P5 until at or near the onset of the next release phase 42.

The process of changing from pressure P5 to pressure P6, from pressure P6 to pressure P7, and then from pressure P7 back to pressure P5 in the NRPAP mode of operation of ventilator 2 is repeated for each ventilation cycle 28 applied to patient 10, which desirably occurs independent of each breath cycle of patient 10. However, if desired, each ventilation cycle in the NRPAP mode of operation can be synchronized with each breath of patient 10 whereupon, it is envisioned that patient 10 would exhale during release phase 42 and/or just prior to the onset of release phase 42, and inhale during delivery phase 40 and exhale during the exhalation phase 46. However, this is not to be construed as limiting the invention.

Comparing FIGS. 2B and 4B, it can be seen that in the NRPAP mode of operation, the onset of release phase 42 is delayed until just before the next delivery phase 40. This is similar to the APRV mode of operation discussed above where the onset of second period 32 is delayed until just before the next first period 30. Moreover, like the APRV mode of operation, maintaining the flow of fluid at pressure P5 during quiescent phase 44 in the NRPAP mode of operation increases the patient's FRC above the patient's FRC in the conventional mode of operation.

Because the flow of fluid is provided to patient 10 at pressure P7 during delivery phase 40 in the NRPAP mode of operation, it is believed that the volume of fluid introduced into patient 10 in the NRPAP mode of operation during delivery phase 40 will be greater than the volume of fluid introduced into patient 10 during first period 30 in the APRV mode of operation. As a result, it is believed that V_{TF}, i.e., the fresh fluid tidal volume or fresh fluid volume entering the patient, in the NRPAP mode of operation will be greater than V_{TF} in both the APRV mode of operation and the conventional mode of operation. Thus, in the NRPAP mode of operation, it is believed that more fluid will be exchanged in the lungs of patient 10 during each ventilation cycle 28 than in either the APRV mode of operation or the conventional mode of operation.

The area shown in crosshatch in FIG. 4B represents the velocity of fresh fluid that flows into the patient's lungs during delivery phase 40 and corresponds to V_{TF} in FIG. 4C. As shown in FIG. 4C, V_{TF} equals V_{T} in the NRPAP mode of operation. Because providing the flow of fluid at pressure P5 during exhalation/quiescent phase 46, 44 and at pressure P7 during delivery phase 40 may not be comfortable for some patients 10, it may be necessary to sedate patient 10 when ventilator 2 is operated in the NRPAP mode of operation. As can be seen, the present invention is a method of ventilating patient 10 that increases the volume of fresh fluid provided to patient 10 and increases the volume of fluid exchanged in the lungs of the patient 10 over ventilating patient 10 with the conventional or APRV modes of operation.

In the embodiment described above, the flow of fluid is described as being pressure limited. That is, during the exhalation/quiescent phase, the release phase, and the delivery phase, the flow of fluid is controlled based on the pressure of the fluid flow. It is to be understood, that the present invention also contemplates controlling the flow of fluid during one or more of these phases based on other characteristics. For example, the present invention also contemplates controlling the flow of fluid based on the flow rate or the volume. When operated in a volume limited mode, for example, the ventilator seeks to maintain the volume of fluid in the patient to a baseline level during the exhalation/quiescent phase, to decrease the volume of fluid in the patient during the release phase below the baseline level, increase the volume of fluid in the patient during the delivery phase above the baseline level, and allow the volume of fluid in the patient to return to the baseline level during the next exhalation phase and to reach the quiescent phase.

When operated in a flow rate limited mode, for example, the ventilator seeks to maintain the flow of fluid in the patient to a baseline level during the exhalation/quiescent phase, to decrease the flow of fluid in the patient during the release phase below the baseline level, e.g., to remove some used gas from the patient's airways or lungs, increase the flow of fluid in the patient during the delivery phase above the baseline level, and allow the flow of fluid in the patient to return to the baseline level during the next exhalation phase towards the quiescent phase.

The present invention also contemplates that the duration of the quiescent phase, the release phase, the delivery phase, and the exhalation phase, can be controlled based on time, rather than on the pressure, flow, or volume of fluid delivered to the patient. For example, the ventilator can be programmed to initiate the release phase at a predetermined period of time following the end of the previous delivery phase. The duration of the release phase can be set to a predetermined period of time. Similarly, the duration of the delivery phase can be set to a predetermined period of time. Of course, using timed based control of the pressure, flow, or volume changes is best suited for a patient that is not spontaneously breathing.

The present invention also contemplates that the shape of the pressure, flow, or volume waveform during the release phase, the delivery phase, and/or the return to the baseline level of the exhalation and quiescent phases following the delivery phase can be controlled so as to have a specific profile or contour. Conversely, the shape of the pressure, flow, or volume waveform during the release phase, the delivery phase, and/or the return to the baseline level of the exhalation/quiescent phase following the delivery phase can be uncontrolled. In other words, in a pressure limited mode, the ventilator can seek to hit a target pressure, such as the baseline, release, and peak pressure during the quiescent phase, the release phase, and the delivery phase, respectively, but the specific shape of the pressure curve can be left uncontrolled. In which case, shape of the pressure curve will be dictated by the mechanical capabilities of the ventilator, the patient effort, and the respiratory mechanics of the patient, such as his or her respiratory compliance and resistance. It is to be further understood that the shape of the pressure, flow, or volume waveforms can be controlled during one phase and uncontrolled during another phase within the same breath cycle.

It is to be further understood that the present invention contemplates controlling the level of the pressure (P5, P6, P7), the flow, or the volume delivered during the quiescent phase, the release phase, the delivery phase, and the exhalation phase on an active basis. That is, the level of the pressure, the flow, or the volume delivered during the quiescent phase, the release phase, the delivery phase, and the exhalation phase can automatically titrated by the ventilator system based on the monitored condition of the patient and/or the ventilator system. Techniques for automatically adjusting the pressure of a flow of gas delivered to a patient are well known. The present invention contemplates using these conventional autotitration techniques for setting the level of the pressure, the flow, or the volume delivered during the quiescent phase, the release phase, the delivery phase, and the exhalation phase. This can be done on an ongoing basis, such as on a breath-by-breath basis, or less frequently.

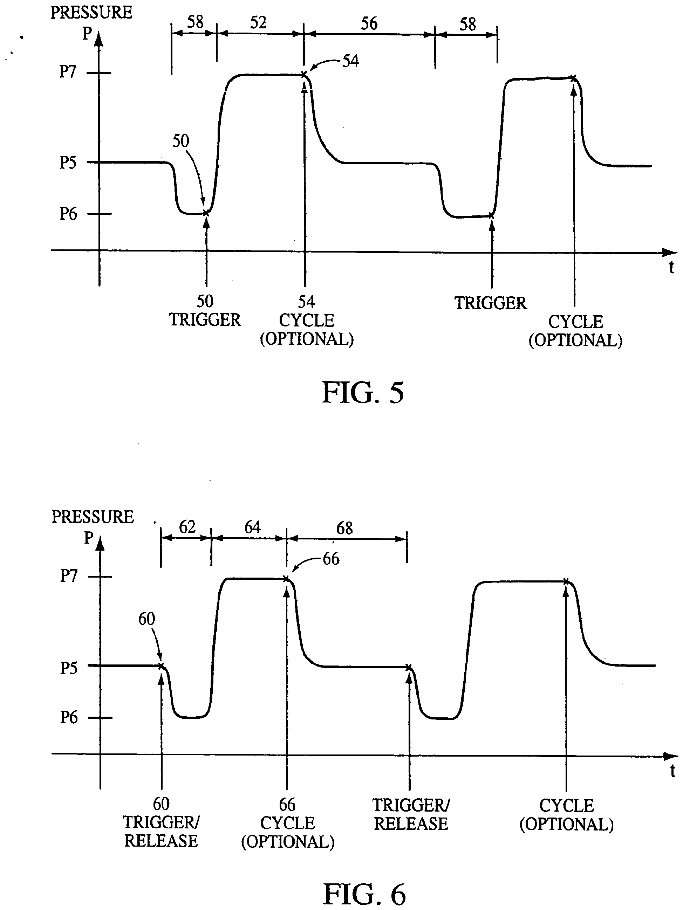

As noted above, the present invention contemplates that each ventilation cycle in the NRPAP mode of operation can be synchronized with each patient breath. There are several techniques by which the pressure delivery provided by the NRPAP mode of ventilation can be synchronized with the patient's spontaneous respiration. These techniques are discussed below with reference to FIGS. 5-7, each of which illustrates an exemplary pressure waveform provided to the patient by the pressure generating system. As used herein, the term "trigger" refers to the transition from the expiratory to the inspiratory phase of the breathing cycle, and the term "cycle" refers to the transition from the inspiratory to the expiratory phase of the breathing cycle. The term "trigger" can also refer to transition to release phase initiated by the inspiratory effort or signal. The term "cycle" can also refer to transition to release phase initiated by expiratory effort or signal. It should be noted that the terms "trigger" and "cycle" as used herein, refer to the transition from one phase to the next, and are not intended to imply that the ventilation system necessarily adjusts or changes the pressure, flow, or volume of gas delivered to the patient upon detection of a trigger or cycle event.

FIG. 5 illustrates an NRPAP mode of ventilation in which the patient's spontaneous inspiratory effort is a trigger point 50, and initiates a delivery phase 52. The duration of the delivery phase can be cycled based on the patient's expiratory effort, time, flow, volume, pressure, or any combination thereof. That is, if the patient attempts to exhale, this effort can be sensed using any conventional technique and the ventilator cycles at point 54 from delivery phase 52 to an exhalation/quiescent phase 56. This cycle can also occur if a predetermined period of time has elapsed, a threshold flow rate is sensed, a threshold volume is reached, a threshold pressure is reached, or any combination thereof. This is why the cycle points in FIG. 5 are indicated as being optional, i.e., the ventilator need not use the patient's expiratory effort as a cycle event.

The duration of exhalation/quiescent phase 56 can be controlled based on the patient's effort, time, flow, volume, or pressure. For example, the present invention contemplates waiting a predetermined period of time after cycle point 54 and thereafter initiating a release phase 58. It is to be understood that the initiation of release phase 58, i.e., the control of the duration of exhalation/quiescent phase 56, can be based on monitored parameters other than being purely time based. For example, the initiation of release phase 58 can occur if the flow, pressure, volume, or any combination thereof reach predetermined thresholds. In this embodiment, the system remains in the release phase until a trigger event occurs, for example, the patient spontaneously initiates an inspiration. The trigger causes the system to enter the delivery phase and the process described above repeats.

FIG. 6 illustrates an NRPAP mode of ventilation in which the patient's spontaneous inspiratory effort is a trigger point 60, and initiates a release phase 62. That is, just as the patient is beginning to inspire, this is sensed using any conventional technique and release phase 62 is initiated. The duration of the release phase can be controlled based on the patient's effort, time, flow, volume, pressure, or any combination thereof. For example, if the patient attempts to further inhale, this inspiratory effort can be sensed using any conventional technique, and the ventilator transitions to a delivery phase 64. The transition from the release phase 62 to delivery phase 64 can also occur if a predetermined period of time has elapsed, a threshold flow rate is sensed, a threshold volume is reached, a threshold pressure is reached, or any combination thereof.

The duration of delivery phase 64 can be cycled based on the patient's expiratory effort, time, flow, volume, pressure, or any combination thereof. That is, if the patient attempts to exhale once the delivery phase has begun, this expiratory effort can be sensed using any conventional technique, and the ventilator cycles at point 66 to exhalation/quiescent phase 68. This cycle can also occur if a predetermined period of time has elapsed, a threshold flow rate is sensed, a threshold volume is reached, a threshold pressure is reached, or any combination thereof. In this embodiment, the system remains in exhalation/quiescent phase 68 until the next trigger event occurs, for example, when the patient spontaneously initiates an inspiration. This next trigger causes the system to enter the release phase and the process described above repeats.

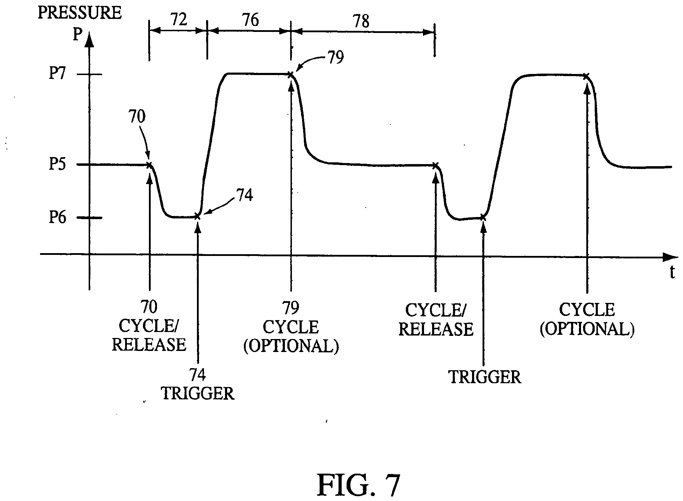

FIG. 7 illustrates an NRPAP mode of ventilation in which the release phase is synchronized with the patient's spontaneous expiratory cycle. At a cycle point 70, the patient begins exhaling and the ventilator transitions from a previous exhalation phase to a release phase 72. Sensing the cycle point is accomplished using any conventional technique. The duration of release phase 72 is controlled based on the patient's effort, time, flow, volume, pressure, or any combination thereof. For example, if the patient attempts to inhale at point 74, this becomes a trigger point 74 causing the ventilator to transition to a delivery phase 76.

The duration of delivery phase 76 can be controlled based on time, flow, volume, pressure, or any combination thereof. That is, if a predetermined period of time has elapsed, a threshold flow rate is sensed, a threshold volume is reached, a threshold pressure is reached, or any combination thereof, the system ends the delivery phase and transitions to an exhalation/quiescent phase 78. In this embodiment, the system remains in exhalation/quiescent phase 78 until the next cycle event occurs, for example, when the patient spontaneously initiates an expiration. This next cycle causes the system to enter the release phase and the process described above repeats.

It should be noted that the transition from release phase 72 to delivery phase 76 need not be based on the patient's own inspiratory effort. In which case, if the patient attempt to inspire, the inspiratory effort would have no impact on the operation of the ventilation system. Thus, the triggers shown in FIG. 7 represent a pressure transition, but need not be based on the patient's inspiratory effort.

It should be emphasized that the patient will be able to breath spontaneously (assisted or not assisted, supported or not supported) during all of the NRPAP phases. In other embodiments of the present invention, the patient will not be able to breath spontaneously in some or all of the NRPAP phases.

It should also be emphasized that any or all of the NRPAP phases can be very short or very long in relation to the duration of inspiration or expiration of normal breathing. In another implementations of the NRPAP mode of operation, the NRPAP phases will or will not be related to the respiratory cycles. For example, the pressure can be changed in accordance to the NRPAP principle to control the Functional Residual Capacity (FRC) and allow the patient to breath at different FRC levels for periods of time.

The NRPAP mode of operation invention has been described above in the context of using this technique to increase the amount of fresh fluid that is introduced into the lungs of the patient by controlling the volume, pressure, or flow of fluid using tri-level flow/pressure variations. Typically, this is done to augment or replace a patient's own ventilatory effort. It is to be understood, however, that the present invention contemplates using the NRPAP mode of operation for therapeutic purposes that are not related to ventilation or that are not related to the use of a ventilator. Several examples of these other applications for the NRPAP mode of operation are discussed below.

The NRPAP pressure applying technique can be used in the context of applying external pressure to the surface of the patient. For example, the NRPAP type of variations can be used to control the pressure applied externally to a patient during resuscitation or during physiotherapy. In this embodiment, the NRPAP mode of operation is imposed on the pressure variations introduced onto the surface of the patient. The NRPAP technique can be used to control blood pressure or to control the activity of the heart, with or without using the respiratory system. A fluid pump can be operated according to the NRPAP mode of operation. Such an NRPAP pump would augment or replace the function of the heart, for example. The NRPAP mode of operation can also be used to apply pressure to a patient's circulatory or other physiological systems or portions thereof, such as the arteries, blood vessels or other vessels. In short, the NRPAP or tri-level mode of applying pressure support, including the pressure release phase, can be applied generally in many areas, for instance in physiotherapy, massage, leg or arm pressure cuffs, and abdominal and/or bowl movement stimulation. It is to be understood that these are merely examples of other uses for the NRPAP mode of operation in areas outside the realm of ventilation. The present invention in not intended to be limited to these specific examples for the use of the NRPAP mode of operation.

The present invention has been described with reference to the preferred embodiment. Obvious modifications and alterations will occur to others upon reading and understanding the preceding detailed description. It is intended that the present invention be construed as including all such modifications and alterations insofar as they come within the scope of the appended claims.

## Claims

1. A medical ventilator (2) comprising:
means adapted to deliver fluid to a patient (10) during an exhalation/quiescent phase (44, 46), in which a pressure, a flow, or a volume of fluid is provided at a baseline level;
means adapted to release the flow of fluid from such a patient (10) during a release phase (42), in which the pressure, the flow, or the volume of fluid is decreased from the baseline level to a release level that is less than the baseline level;
means adapted to increase the pressure, the flow, or the volume of fluid delivered to such a patient (10) during a delivery phase (40), in which the pressure, the flow, or the volume of fluid is increased from the release level to a peak level above the baseline level; and
means adapted to allow the pressure, the flow, or the volume of fluid to return from the peak level to the baseline level,**characterized in that** the release phase (42) follows the exhalation/quiescent phase (44, 46) and the delivery phase (40) follows the release phase (42), wherein the baseline level of the flow of fluid delivered during the exhalation/quiescent phase (44, 46) is a pressure ranging from 5-30 cm H₂O, wherein the release level of the flow of fluid delivered during the release phase (42) is a pressure ranging from 0-15 cm H₂O, and wherein the peak level of the flow of fluid delivered during the delivery phase (40) is a pressure ranging from 15-60 cm H₂O.

2. The ventilator (2) of claim 1, wherein the exhalation/quiescent phase (44, 46), the release phase (42), the delivery phase (40), or any combination thereof is entered (a) independent of such a patient's (10) spontaneous respiration, or (b) in synchronization with such a patient's (10) spontaneous respiration.

3. The ventilator (2) of claim 1, wherein the means for increasing the pressure, the flow, or the volume of fluid delivered to such a patient (10) during the delivery phase (40) are adapted to be synchronized with such a patient's (10) inspiration.

4. The ventilator (2) of claim 1, wherein the means for increasing the pressure, the flow, or the volume of fluid delivered to such a patient (10) during the delivery phase (40) is pressure, volume, or flow limited.

5. The ventilator (2) of claim 1, wherein the means for delivering fluid to a patient (10) during the exhalation/quiescent phase (44, 46) are adapted to control a duration of the exhalation/quiescent phase (44, 46) based on time, wherein the means for releasing the flow of fluid from such a patient (10) during a release phase (42) are adapted to control a duration of the release phase (42) based on time, and wherein the means for increasing the flow of fluid delivered to such a patient (10) during a delivery phase (40) are adapted to control a duration of the delivery phase (40) is controlled based on time.

6. A medical ventilator (2) according to claim 1, comprising:
a patient circuit;
a pressure generator for delivering pressurized fluid to a patient (10) via the patient circuit; and
means for controlling the pressure generator to (a) supply the fluid to the patient (10) at first pressure at said baseline level (P5), (b) supply the fluid to the patient (10) at a second pressure at said release level (P6) less than the first pressure, and (c) supply the fluid to the patient (10) at a third pressure at said peak level (P7) greater than the first pressure.

7. The medical ventilator (2) of claim 6, wherein the means for controlling is responsive to the patient's (10) breathing for:
supplying the fluid to the patient (10) at the first or second pressures when the patient (10) is exhaling; and
supplying the fluid to the patient (10) at the third pressure when the patient (10) is inhaling.

8. The medical ventilator (2) of claim 6, wherein the means for controlling are adapted to control the pressure generator to cyclically supply the fluid to the patient (10) at the first, second and third pressures independent of the patient's (10) breathing.

9. The ventilator (2) of claim 6, wherein the means for controlling control the pressure that enters the exhalation/quiescent phase (44, 46), the release phase (42), the delivery phase (40), or any combination thereof (a) independent of such a patient's (10) spontaneous respiration, or (b) in synchronization with such a patient's (10) spontaneous respiration.

10. The ventilator (2) of claim 6, wherein the means for controlling are the pressure controlling means that pressure limits, volume limits, or flow limits, the pressure, flow or volume increase during the delivery phase (40).

11. The ventilator (2) of claim 6, wherein the means for controlling are pressure controlling means that are adapted to control a duration of the exhalation/quiescent phase (44, 46), the release phase (42), or the delivery phase (40) based on time.

## Patentansprüche

1. Medizinisches Beatmungsgerät (2), umfassend:
Mittel, die ausgelegt sind, um ein Fluid an einen Patienten (10) während der Ausatmungs-/Ruhephase (44, 46) abzugeben, in der ein Druck, ein Strom oder ein Volumen von Fluid auf einem Grundpegel bereitgestellt ist;
Mittel, die ausgelegt sind, um den Strom von Fluid von einem solchen Patienten (10) während einer Freigabephase (42) freizugeben, in welcher der Druck, der Strom oder das Volumen von Fluid von dem Grundpegel auf einen Freigabepegel verringert wird, der kleiner als der Grundpegel ist;
Mittel, die ausgelegt sind, um den Druck, den Strom oder das Volumen von Fluid, das an einen solchen Patienten (10) während einer Abgabephase (40) abgegeben wird, zu erhöhen, wobei der Druck, der Strom oder das Volumen von Fluid von dem Freigabepegel zu einem Spitzenpegel oberhalb des Grundpegels erhöht wird; und
Mittel, die ausgelegt sind, um dem Druck, dem Strom oder dem Volumen von Fluid zu ermöglichen, von dem Spitzenpegel zu dem Grundpegel zurückzukehren, **dadurch gekennzeichnet, dass** die Freigabephase (42) der Ausatmungs-/Ruhephase (44, 46) folgt und die Abgabephase (40) der Freigabephase (42) folgt, wobei der Grundpegel des Stroms von Fluid, das während der Ausatmungs-/Ruhephase (44, 46) abgegeben wird, ein Druck im Bereich von 5 bis 30 cm H₂O ist, wobei der Freigabepegel des Stroms von Fluid, das während der Freigabephase (42) abgegeben wird, ein Druck im Bereich von 0 bis 15 cm H₂O ist und wobei der Spitzenpegel des Stroms von Fluid, das während der Abgabephase (40) abgegeben wird, ein Druck im Bereich von 15 bis 60 cm H₂O ist.

2. Beatmungsgerät (2) nach Anspruch 1, wobei die Ausatmungs-/Ruhephase (44, 46), die Freigabephase (42), die Abgabephase (40) oder eine beliebige Kombination davon (a) unabhängig von einer spontanen Atmung eines solchen Patienten (10) oder (b) in Synchronisation mit der spontanen Atmung eines solchen Patienten (10) eingegeben werden.

3. Beatmungsgerät (2) nach Anspruch 1, wobei die Mittel zum Erhöhen des Drucks, des Stroms oder des Volumens von Fluid, das an einen solchen Patienten (10) während der Abgabephase (40) abgegeben wird, dazu ausgelegt sind, mit der Einatmung eines solchen Patienten (10) synchronisiert zu sein.

4. Beatmungsgerät (2) nach Anspruch 1, wobei das Mittel zum Erhöhen des Drucks, des Stroms oder des Volumens von Fluid, das an einen solchen Patienten (10) während der Abgabephase (40) abgegeben wird, druck-, volumen- oder strombegrenzt ist.

5. Beatmungsgerät (2) nach Anspruch 1, wobei die Mittel zum Abgeben von Fluid an einen Patienten (10) während der Ausatmungs-/Ruhephase (44, 46) dazu ausgelegt sind, eine Dauer der Ausatmungs-/ Ruhephase (44, 46) zeitabhängig zu steuern, wobei die Mittel zum Freigeben des Strom von Fluid von einem solchen Patienten (10) während einer Freigabephase (42) dazu ausgelegt sind, eine Dauer der Freigabephase (42) zeitabhängig zu steuern, und wobei die Mittel zum Erhöhen des Stroms von Fluid, das an einen solchen Patienten (10) während einer Abgabephase (40) abgegeben wird, dazu ausgelegt sind, eine Dauer der Abgabephase (40) zeitabhängig zu steuern.

6. Medizinisches Beatmungsgerät (2) nach Anspruch 1, umfassend:
ein Patientenkreislauf;
einen Druckerzeuger zum Abgeben von druckbeaufschlagtem Fluid an einen Patienten (10) über den Patientenkreislauf; und
Mittel zum Steuern des Druckerzeugers, um (a) das Fluid an den Patienten (10) bei einem ersten Druck auf dem Grundpegel (P5) abzugeben, (b) das Fluid an den Patienten (10) mit einem zweiten Druck auf dem Freigabepegel (P6) abzugeben, der kleiner als der erste Druck ist, und (c) das Fluid an den Patienten (10) bei einem dritten Druck auf dem Spitzenpegel (P7) abzugeben, der größer als der erste Druck ist.

7. Medizinisches Beatmungsgerät (2) nach Anspruch 6, wobei Steuermittel auf die Atmung des Patienten (10) reagiert zum:
Abgeben des Fluids an den Patienten (10) bei dem ersten oder zweiten Druck, wenn der Patient (10) ausatmet; und
Abgeben des Fluids an den Patienten (10) bei dem dritten Druck, wenn der Patient (10) einatmet.

8. Medizinisches Beatmungsgerät (2) nach Anspruch 6, wobei das Mittel zum Steuern dazu ausgelegt ist, den Druckerzeuger zu steuern, um das Fluid zyklisch an den Patienten (10) bei dem ersten, zweiten und dritten Druck unabhängig von der Atmung des Patienten (10) abzugeben.

9. Beatmungsgerät (2) nach Anspruch 6, wobei das Mittel zum Steuern des Drucks, der in die Ausatmungs-/Ruhephase (44, 46), die Freigabephase (42), die Abgabephase (40) oder eine beliebige Kombination davon (a) unabhängig von der spontanen Atmung eines solchen Patienten (10) oder (b) synchron mit der spontanen Atmung eines solchen Patienten (10) eintritt.

10. Beatmungsgerät (2) nach Anspruch 6, wobei die Steuermittel die Drucksteuermittel, die Druckgrenzwerte, Volumengrenzwerte oder Durchflussgrenzwerte sind, bei denen der Druck, der Strom oder das Volumen während der Abgabephase (40) ansteigen.

11. Beatmungsgerät (2) nach Anspruch 6, wobei die Steuermittel Drucksteuermittel sind, die dazu ausgelegt sind, eine Dauer der Ausatmungs-/Ruhephase (44, 46), der Freigabephase (42) oder der Abgabephase (40) zeitabhängig zu steuern.

## Revendications

1. Ventilateur médical (2) comprenant :
un moyen adapté pour distribuer un fluide à un patient (10) durant une phase d'expiration/repos (44, 46), dans laquelle une pression, un flux, ou un volume de fluide est fourni à un niveau de base ;
un moyen adapté pour libérer le flux de fluide depuis un tel patient (10) durant une phase de libération (42), dans laquelle la pression, le flux, ou le volume de fluide diminue depuis le niveau de base à un niveau de libération qui est inférieur au niveau de base ;
un moyen adapté pour augmenter la pression, le flux, ou le volume de fluide distribué à un tel patient (10) durant une phase de distribution (40), dans laquelle la pression, le flux, ou le volume de fluide augmente depuis le niveau de libération à un niveau de crête au-dessus du niveau de base; et
un moyen adapté pour permettre à la pression, au flux ou au volume de fluide de revenir depuis le niveau de crête au niveau de base, **caractérisé en ce que** la phase de libération (42) suit la phase d'expiration/de repos (44, 46) et la phase de distribution (40) suit la phase de libération (42), dans lequel le niveau de base du flux de fluide distribué durant la phase d'expiration/de repos (44, 46) est une pression comprise dans la plage de 5 à 30 cm H₂O, dans lequel le niveau de libération du flux de fluide distribué durant la phase de libération (42) est une pression comprise dans la plage de 0 à 15 cm H₂O et dans lequel le niveau de crête du flux de fluide distribué durant la phase de distribution (40) est une pression comprise dans la plage de 15 à 60 cm H₂O.

2. Ventilateur (2) selon la revendication 1, dans lequel on entre dans la phase d'expiration/de repos (44, 46), la phase de libération (42), la phase de distribution (40), ou toute combinaison de celles-ci (a) indépendamment de la respiration spontanée d'un tel patient (10), ou (b) en synchronisation avec la respiration spontanée d'un tel patient (10).

3. Ventilateur (2) selon la revendication 1, dans lequel le moyen pour augmenter la pression, le flux ou le volume de fluide distribué à un tel patient (10) durant la phase de distribution (40) est adapté pour être synchronisé avec l'inspiration d'un tel patient (10).

4. Ventilateur (2) selon la revendication 1, dans lequel le moyen pour augmenter la pression, le flux ou le volume de fluide distribué à un tel patient (10) durant la phase de distribution (40) est limité en pression, en volume ou en flux.

5. Ventilateur (2) selon la revendication 1, dans lequel le moyen pour distribuer un fluide à un patient (10) durant la phase d'expiration/de repos (44, 46) est adapté pour commander une durée de la phase d'expiration/de repos (44, 46) sur la base du temps, dans lequel le moyen pour libérer le flux de fluide depuis un tel patient (10) durant une phase de libération (42) est adapté pour commander une durée de la phase de libération (42) sur la base du temps, et dans lequel le moyen pour augmenter le flux de fluide distribué à un tel patient (10) durant une phase de distribution (40) est adapté pour commander une durée de la phase de distribution (40) sur la base du temps.

6. Ventilateur médical (2) selon la revendication 1, comprenant :
un circuit patient ;
un générateur de pression pour distribuer un fluide sous pression à un patient (10) via le circuit patient ; et
un moyen pour commander le générateur de pression pour (a) apporter le fluide au patient (10) à une première pression audit niveau de base (P5), (b) apporter le fluide au patient (10) à une deuxième pression audit niveau de libération (P6) inférieure à la première pression, et (c) apporter le fluide au patient (10) à une troisième pression audit niveau de crête (P7) supérieure à la première pression.

7. Ventilateur médical (2) selon la revendication 6, dans lequel le moyen de commande répond à la respiration du patient (10) pour :
apporter le fluide au patient (10) à la première ou deuxième pression quand le patient (10) expire ; et
apporter le fluide au patient (10) à la troisième pression quand le patient (10) inspire.

8. Ventilateur médical (2) selon la revendication 6, dans lequel le moyen de commande est adapté pour commander le générateur de pression pour apporter de manière cyclique le fluide au patient (10) aux première, deuxième et troisième pressions indépendamment de la respiration du patient (10).

9. Ventilateur (2) selon la revendication 6, dans lequel le moyen de commande commande la pression qui entre dans la phase d'expiration/de repos (44, 46), la phase de libération (42), la phase de distribution (40), ou toute combinaison de celles-ci (a) indépendamment de la respiration spontanée d'un tel patient (10), ou (b) en synchronisation avec la respiration spontanée d'un tel patient (10).

10. Ventilateur (2) selon la revendication 6, dans lequel le moyen de commande est le moyen de commande de pression qui limite en pression, limite en volume ou limite en flux l'augmentation de pression, de flux ou de volume durant la phase de distribution (40).

11. Ventilateur (2) selon la revendication 6, dans lequel le moyen de commande est un moyen de commande de pression qui est adapté pour commander une durée de la phase d'expiration/de repos (44, 46), de la phase de libération (42), ou de la phase de distribution (40) sur la base du temps.
